Europäisches Patentamt

European Patent Office

Office européen des brevets

(11) Numéro de publication: **0 187 092**
**B1**

(12) **FASCICULE DE BREVET EUROPEEN**

(45) Date de publication du fascicule du brevet: **05.09.90**

(21) Numéro de dépôt: **85402484.1**

(22) Date de dépôt: **13.12.85**

(51) Int. Cl.⁵: **C 12 P 19/06,** C 07 K 7/02,
E 21 B 43/25

(54) **Procédé de production de polysaccharides de type xanthane.**

(30) Priorité: **21.12.84 FR 8419622**

(43) Date de publication de la demande:
**09.07.86 Bulletin 86/28**

(45) Mention de la délivrance du brevet:
**05.09.90 Bulletin 90/36**

(84) Etats contractants désignés:
**AT DE FR GB**

(56) Documents cités:
**EP-A-0 058 364**
**EP-A-0 074 775**
**EP-A-0 098 473**
**GB-A-1 174 322**

**Biotechnology, Vol. 3 (H.Dellweg ed.) 1983, pp.
43-50, 62-64, Verlag Chemie**

**Ullmanns Encyclopädie der technischen
Chemie, Band 10, 1975, Verlag Chemie,
Weinheim,pp. 449-470**

**Biotechn.Bioeng., Vol. XIV, 1972, pp. 379-390
Agr.Biol.Chem., Vol. 33, no. 10, 1969, pp.
1521-1522**

(73) Titulaire: **RHONE-POULENC CHIMIE
25, quai Paul Doumer
F-92408 Courbevoie Cédex (FR)**

(72) Inventeur: **Leproux, Véronique
83, rue d'Aguesseau
F-92100 Boulogne (FR)**
Inventeur: **Peignier, Michel
rue du Joly - Lentilly
F-69210 l'Arbresle (FR)**
Inventeur: **Cros, Patrick
Collectif Champs Persés 1, rue des Monnaies
F-79500 Melle (FR)**
Inventeur: **Beucherie, Jeanine
4, rue Pasteur
F-91300 Massy (FR)**
Inventeur: **Kennel, Yves
2, rue Eloy Ricard
F-79500 Melle (FR)**

(74) Mandataire: **Tavernier, Colette et al
RHONE-POULENC INTERSERVICES Service
Brevets Chimie 25, Quai Paul Doumer
F-92408 Courbevoie Cédex (FR)**

Courier Press, Leamington Spa, England.

**Description**

La présente invention concerne un procédé de production de polysaccharides par fermentation de carbohydrates à l'aide de microorganismes. L'invention concerne plus particulièrement un procédé de fermentation en émulsion.

Les hétéropolysaccharides ou biopolymères obtenus par fermentation d'un hydrate de carbone sous l'action de bactéries du genre Xanthomonas, Arthrobacter, de champignon appartenant au genre Sclerotium ou d'autres microorganismes du même genre, ont trouvé de nombreuses applications industrielles en raison de leurs propriétés épaississantes et viscosifiantes.

La production de gomme xanthane par fermentation aérobie en milieu aqueux a été décrite dans de nombreux brevets; voir par exemple les brevets américans 3 000 790, 3 020 206, 3 391 060, 3 433 708, 4 119 546, 4 154 654, 4 296 203, 4 377 637 et le brevet français N° 2 414 555.

A l'issue du processus de production, le bouillon de fermentation peut contenir environ 15 à 50 g/litre de polysaccharide. Industriellement, il est très difficile de dépasser une concentration de 30 à 35 g/litre sans mettre en oeuvre des mesures spéciales. En effet, l'augmentation de viscosité du mileu réactionnel au fur et à mesure de la formation du polysaccharide relantit le transfert d'oxygène et freine la fermentation. Même si les réacteurs sont équipés de moyens d'agitation puissants, qui sont coûteux en énergie, il est très difficile d'assurer une aération et un brassage suffisants de la masse réactionnelle qui permettraient un accroissement de concentration en polymère.

Dans le but d'accroître la concentration en gomme, il a récemment été proposé de réaliser la fermentation sous forme d'une émulsion ou dispersion du milieu nutritif aqueux dans une huile de manière à réduire la viscosité du moût et à favoriser le transfert d'oxygène: demandes de brevet européen 00 58364, 00 74775 et 00 98473.

Il apparaît cependant préférable de réaliser une première étape de croissance des microorganismes dans le milieu nutritif essentiellement aqueux avant l'introduction de l'huile et la constitution de l'émulsion eau dans huile. Lorsque l'émulsion eau dans l'huile est formée, il devient difficile de réguler le pH de la phase aqueuse dispersée et la formation de sous-produits acides risque de provoquer une inhibition de la croissance du microorganisme. On peut certes travailler en milieu tamponné mais la présence du tampon peut être défavorable à la qualité des solutions obtenues. Pars ailleurs, les émulsions eau dans huile stabilisées ne se diluent que très difficilement, ce qui est un inconvénient si l'on envisage d'utiliser directement ces émulsions par exemple pour l'exploitation des gisements pétroliers.

Le présente invention a pour but de proposer un procédé de fermentation permettant de réaliser aisément la croissance des microorganismes en milieu émulsionné.

L'invention a également pour but la réalisation d'émulsions stables de biopolymères pouvant contenir jusqu'à environ 60% de biopolymère.

Le présent procédé de production de polysaccharides épaississants par fermentation d'un milieu nutritif aqueux contenant un hydrate de carbone à titre de source hydrocarbonée au moyen de microorganismes est caractérisé en ce que l'on disperse une huile dans le milieu aqueux de manière à former une émulsion huile dans eau au sein de laquelle est réalisé la fermentation.

La phase aqueuse continue peut être choisie parmi tous les milieux de fermentation décrits dans le littérature. Des détails peuvent être trouvés par exemple dans les brevets précité US 3 391 060, US 3 443 708 et FR 2 414 555. Le milieu conventionnel contient une source hydrocarbonée qui peut être un sucre ou autre hydrate de carbone, une source d'azote organique et/ou minérale, ainsi que les traces d'éléments et si nécessaire des facteurs de croissance. La concentration en sucre, tel que glycose ou saccharose peut varier entre 10 et 100 g/litre et peut même atteindre 150 à 200 g/litre.

Le microorganisme agent de la fermentation est choisi parmi les bactéries qui fermentent les hydrates de carbone telles que cells décrites dans BERGEY'S MANUAL OF DETERMINATE BACTERIOLOGY (8e édition — 1974 — Williams N. Wilkins C° Baltimore) et par exemple *Xanthomonas begoniae, Xanthomonas campestris, Xanthomonas carotae, Xanthomonas hedera, Xanthomonas incanae, Xanthomonas malvacearum, Xanthomonas papaveri cola, Xanthomonas phaseoli, Xanthomonas pisi, Xanthomonas vasculorum, Xanthomonas vericatoria, Xanthomonas vitians, Xanthomonas pelargonii*; les bactérias du genre *Arthrobacter* et plus particulièrement les espèces *Arthrobacter stabilis, Arthrobacter viscosus*; du genre *Erwinia*; du genre *Azotobacter* et plus particulièrement l'espèce *Azotobacter indicus*; du genre *Agrobacterium* et plus particulièrement les espèces *Agrobacterium radiobacter, Agrobacterium rhizogenes, Agrobacterium tumefaciens.* Peuvent aussi être utilisés les champignons appartenant au genre *Sclerotium.*

On préfère utiliser parmi ces microorganismes *Xanthomonas campestris.*

En ci qui concerne la phase huileuse, on peut utiliser toute huile minérale ou végétale non miscible à l'eau, par exemple les isoparaffines, le kérosène déodorisé, les hydrocarbures ayant un point d'ébullition élevé, de préférence supérieur à 150—200°C, les huiles de colza, de soja, de maïs de tournesol, d'arachide etc. . . . Il est souhaitable que la proportion de phase huileuse représente moins de 30% du milieu total, de préférence 1 à 18% et de manière encore plus préférée 3 à 16%.

La dispersion et la stabilisation de la phase huileuse dans la phase aqueuse sont favorisées par la présence d'agents tensio-actifs, de préférence non ioniques, dont la valeur HLB est préférablement inférieure à 11 et plus préférentiellement comprise entre 6 et 11, cette valeur pouvant être obtenue à l'aide d'un seul tensio-actif ou d'un mélange de tensio-actifs. Le choix et la quantité de tensio-actif nécessair à l'obtention d'une phase continue aqueuse est facilement déterminable par l'homme de métier en fonction de l'huile choisie et de sa concentration dans le milieu.

Comme agent tensio-actif non ionique, on peut faire appel de manière générale à des composés obtenus par condensation d'oxyde d'alcoylène avec un composé organique aliphatique ou alcoyl-aromatique. Des agents tensio-actifs appropriés qui n'inhibent pas la croissance des micro-organismes sont les alcoylphénols polyoxyéthylénés, les alcools polyoxyéthylénés, les acides gras polyoxyéthylénés, les triglycérides polyoxyéthylénés, les esters polyoxyéthylénés de sorbitan et d'acides gras.

Tous les agents tensio-actifs peuvent être utilisés seuls ou en mélange entre eux. On peut introduire tout ou partie du/des tensio-actif(s), selon leur affinité, dans la phase huileuse ou la phase aqueuse.

Pour la mise en oeuvre du procédé, il est conseillé de stériliser séparément la phase huilleuse et la phase aqueuse. Après stérilisation et refroidissement, la phase huileuse peut être mélangée dans le phase aqueuse contenue dans le fermenteur ou bien chacune des phases peut être introduite en continu dans le fermenteur sous agitation. Une émulsion stable se forme immédiatement. L'ensemencement et la fermentation sont ensuite réalisés de manière conventionnelle sous aération et agitation. Au cours de la fermentation, le pH peut être maintenu à la valeur optimale par injection d'une solution alcaline ou ammoniacale. Il est également possible d'effectuer périodiquement ou en continu des apports de milieu nécessaire à la croissance.

A l'issue de la fermentation et selon les applications envisagées, l'émulsion peut être utilisée telle quelle, ou bien la phase aqueuse peut être séparée de la phase huileuse par destabilisation de manière connue en soi et la moût utilisé à l'état brut, ou bien encore le polysaccharide peut être précipité par exemple par addition d'un alcool inférieur comme l'isopropanol avec ou sans l'aide d'un sel minéral. Le polysaccharide précipité est séparé, lavé de l'huile adsorbée sur les fibres, puis séché et éventuellement broyé. On obtient ainsi une poudre prête à l'emploi.

Selon un aspect particulier du procédé de l'invention, l'émulsion obtenue après achèvement du processus de fermentation est concentrée par élimination d'eau jusqu'à obtention d'une émulsion contenant 8 à 60% et de préférence 15 à 60% en poids de polysaccharides par rapport au poids de l'émulsion. Dans cet aspect de l'invention, la quantité d'huile mise en oeuvre pour la fermentation doit être calculée en fonction de la teneur en matière sèche en fin de fermentation. Il est préférable que la quantité d'huile dans l'émulsion finale ne dépasse pas 45% du milieu total.

L'élimination de l'eau peut être réalisée par exemple par évaporation ou distillation éventuellement sous pression partielle. Il est possible d'éliminer une partie de la phase huileuse en mélange azéotropique ou non avec l'eau. On peut encore opérer par ultrafiltration en utilisant des techniques et équipements conventionnels à condition évidemment de choisir une membrane poreuse hydrophile dont les pores sont suffisamment petits pour prévenir le passage du biopolymère à travers la membrane.

L'ultrafiltration donne l'assurance de conserver les émulsions en phase continue aqueuse. La concentration par ultrafiltration a encore pour avantage de maintenir les teneurs en sels minéraux de la phase aqueuse à des valeurs voisines de celles présentes dans le moût d'origine, quelle que soit la teneur en polymère.

En variante, on peut concentrer l'émulsion d'abord par ultra-filtration, et ensuite par une autre opération, par exemple évaporation ou distillation.

Sans se départir de l'étendue de l'invention, on peut adjoindre aux émulsions obtenues des bactéricides, des enzymes ou tout autre additif destiné à une utilisation particulière à condition que cet additif ne nuise pas à la stabilité de l'émulsion.

Les avantages du procédé, par rapport au système en émulsion inverse sont notamment:

La possibilité de réaliser la croissance des microorganismes dans le milieu émulsionné avec une vitesse réactionnelle normale. La croissance du microorganisme n'est pas perturbée par l'émulsion.

Une économie d'énergie au cours de la phase de croissance, le puissance dissipée pour agiter le milieu étant plus faible.

La possibilité de réguler facilement le pH de la phase aqueuse.

La possibilité de concentrer les émulsions sans inversion de phases et sans précipitation du polysaccharide.

Les émulsions huile dans eau obtenues selon le procédé de l'invention sont utiles dans toutes les applications requérant des fluides aqueux viscosifiés comme l'industrie du bâtiment, de la peinture, des cosmétiques, du phytosanitaire, du pétrole, etc. . . . Elles ont pour avantage de pouvoir être miss en oeuvre directement pour la préparation de solutions aqueuses diluées sans qu'il soit nécessaire de passer par une phase d'inversion. Elles se dispersent très bien dans l'eau, leur vitesse de dissolution étant au moins égale à celle du moût d'origine. Elles restent pompables et stables dans un intervalle de concentration très large. Leur viscosité est substantiellement inférieure à celle d'une solution aqueuse contenant une quantité équivalente de biopolymère de sorte qu'il est possible d'avoir des émulsions coulables ou pompables avec des concentrations élevées en polysaccharides dans la phase aqueuse.

Du fait de tous ces avantages, les émulsions de l'invention sont particulièrement appropriées pour être mises en oeuvre sur un champ pétrolifère et pour préparer des fluides aqueux destinés à la récupération assistée du pétrole.

Les exemples suivants sont donnés à titre illustratif et non limitatif de l'invention.

3

# EP 0 187 092 B1

Dans tous les exemples, on procédé de la manière générale suivante:

La solution nutritive aqueuse est chargée dans le fermenteur et stérilisée.

L'huile contenant s'il y a lieu le(s) tensio-actif(s) est stérilisée et versée sons agitation dans la solution aqueuse contenue dans le fermenteur à température ambiante.

Le milieu est ensemencé avec une préculture de Xanthomonas campestris sur bouillon nutritif MY saccharose à 10 g/l.

## Exemple 1

Dans un fermenteur de 20 litres, on ensemence avec 210 g de la préculture, 14 litres d'une émulsion H/E stérile contenant en volume 6,25% de phase huileuse et 93,75% de phase aqueuse.

Composition de la phase aqueuse:

| | |
|---|---|
| Dextrose | 61 g/l |
| Extrait de levure | 7 g/l |
| $Na_2HPO_4$ | 2,35 g/l |
| $(NH_4)_2\ PO_4$ | 2,2 g/l |
| $MgSO_4, 7H_2O$ | 0,35 g/l |

Composition de la phase huileuse:

| | |
|---|---|
| Hydrocarbure aliphatique désaromatisé (Exsol D 100® commercialisé par Esso Chimie) | 70% en poids |
| Nonylphénols éthoxylés (Mélange 67/33 de Cemulsol NP4® et Cemulsol NP 17® — Marques commerciales de la Société SFOS) Valeur HLB: 11 | 30% en poids |

Conditions de fermentation:

| | | |
|---|---|---|
| Temperature | 28°C | |
| pH régulé à 7 par addition de soude | | |
| Agitation | 400 rpm | |
| Aération de | 0 à 17 heurs | 0,24 VVM |
| | 17 à 23 heures | 0,48 VVM |
| | 23 à 90 heures | 0,98 VVM |
| Fermentation jusqu'à épuisement total du sucre | | |
| Durée: 90 heures. | | |

L'évolution de la population et la résistivité du milieu au cours du temps sont indiquées dans le Tableau 1.

La quantité de matière active obtenue est de 425 g (30,3 g/kg) soit un rendement par rapport au sucre de 56%.

La viscosité de l'émulsion en fin de fermentation est de 8,1 Pa.s (viscosité Brookfield 20°C — Aiguille n° 4—30 t/mn).

4

## Exemple 2

On renouvelle l'exemple 1 avec un milieu initial composé de 29% en volume d'hydrocarbures Exsol D 100® et 71% de phase aqueuse ayant la composition suivante:

| | |
|---|---|
| Dextrose | 64 g/l |
| Extrait de levure | 9,24 g/l |
| $Na_2HPO_4$ | 3,08 g/l |
| $(NH_4)_2 PO_4$ | 3,08 g/l |
| $Mg SO_4, 7 H_2O$ | 0,35 g/l |

Aucun agent tensio-actif n'est ajouté.

Les conditions opératoires sont identiques à celles de l'exemple 1, sauf l'aération qui est constamment de 0,98 VVM. La fermentation dure 85 heures, stade auquel il n'y a plus de sucre.

L'évolution de la population et la résistivité du milieu sont données dans le Tableau 1.

On obtient 330 g de matière active, soit un rendement de 51,3% par rapport au sucre. La viscosité de l'émulsion finale est de 10,2 Pa.s (mêmes conditions que Exemple 1).

## Exemple 3

L'émulsion H/E initiale est formée à partir de 14,125 litres de phase aqueuse et 0,875 litres de phase huileuse.

Composition de la phase aqueuse:

| | |
|---|---|
| Saccharose | 85 g/l |
| Extrait de maïs soluble (CSL) | 25,5 g/l |
| $Mg SO_4, 7 H_2O$ | 0,28 g/l |

La phase huileuse contient 70% en poids d'huile de colza et 30% de nonylphénols éthoxylés (CEMULSOL NP4)®.

Valeur HLB: 9

Le milieu est ensemencé comme dans l'exemple 1.

Conditions de fermentation:

| | | |
|---|---|---|
| Température | 28°C | |
| pH régulé à 7 par addition de soude | | |
| Agitation de | 0 à 17 heures | 390 rpm |
| | 17 à 24 heures | 500 rpm |
| | 24 à 84 heures | 600 rpm |
| Aération de | 0 à 17 heures | 0,92 VVM |
| | 17 à 84 heures | 1,4 VVM |

Puisance dissipée moyenne: 6,09 KW/m₃

Au bout de 84 heures, on a produit 51 g de gomme xanthane par kg de milieu. Rendement/saccharose consommé: 64%.

La consistance K (d'après la loi d'Ostwald) du milieu est de 37 pa.s et l'indice de psuedoplasticité n est de 0,195.

Par comparaison, un moût aqueux contenant 48,5 g/kg de polyémère présente une consistance K de 70 Pa.s et un indice n de 0,24.

# EP 0 187 092 B1

On poursuit la fermentation jusqu'à 113 heures. On obtient 66 g/kg de polymère. Rendement/saccharose consommé: 83%.

## Exemples 4 et 5

On réalise deux fermentations dans une émulsion H/E contenant 0,875 litre de phase huileuse et 14,125 litres de phase aqueuse.

La composition du milieu est la suivante:

| | |
|---|---|
| Saccharose | 8% |
| Extrait de maïs soluble (CSL) | 4% |
| Mg SO$_4$, 7 H$_2$O | 0,05% |
| Huile | 3,5% |
| Tensio-actifs | 1,5% |

La phase huileuse est constituée d'huile de colza et d'alcools gras naturels éthoxylés (CEMULSOL AS 5® — Société SFOS) pour l'exemple 4, d'hydrocarbures aliphatiques (EXSOL D 80) et de nonylphénols éthoxylés (mélange 50/50 CEMULSOL NP 4® — CEMULSOL NP 17®) pour l'exemple 5.

Les conditions de fermentation sont les suivantes:

Température: 28°C

| | Aération | Agitation |
|---|---|---|
| 0 - 17 heures | 0,92 VVM | 390 rpm |
| après 17 heures | 1,39 VVM | 500 rpm |
| après 41 heures | 1,39 VVM | 500 rpm (Ex. 4) |
| | | 600 rpm (Ex. 5) |

La fermentation est poursuivie jusqu'à consommation totale du sacharose:

| Résultats : | Exemple 4 | Exemple 5 |
|---|---|---|
| Durée | 68 heures | 80 heures |
| Matière active | 47,4 g/kg | 51 g/kg |
| Rdt/Saccharose | 60 % | 64 % |

## Exemples 6 à 14

A l'aide d'1 ml d'une préculture de Xanthomonas campestris sur bouillon nutritif MY saccharose 10 g/litre, on ensemence 100 ml d'un milieu émulsionné H/E contenu dans un erlenmeyer de 500 ml.

La phase aqueuse commune à tous les exemples est identique à celle de l'exemple 3.

On fait varier la composition de la phase huileuse. Chaque flacon contient 95% en poids de phase aqueuse et 5% en poids de phase huileuse. Les flacons sont agités à l'aide d'un agitateur rotatif tournant à 220 rpm avec une amplitude de 5 cm et incubés à 28°C pendant 89 heures.

Les résultats sont indiqués dans le tableau II.

6

EP 0 187 092 B1

Tableau 1

| Age (heures) | Xanthomonas UFC/ml d'émulsion | | Résistivité $\Omega$.cm | |
| | Ex. 1 | Ex. 2 | Ex.1 | Ex.2 |
|---|---|---|---|---|
| 0 | $1,6 \times 10^8$ | $3,9 \times 10^7$ | 216 | – |
| 17 | $4 \times 10^8$ | $3 \times 10^9$ | 216 | – |
| 23 | $8 \times 10^9$ | $5,4 \times 10^9$ | 370 | 354 |
| 43 | $7 \times 10^8$ | $2 \times 10^{10}$ | 216 | 354 |
| 66 | $4 \times 10^8$ | $1,2 \times 10^{10}$ | 216 | 277 |
| 90 | $1 \times 10^8$ | | 185 | 262 |

TABLEAU 2

| Exemple | | 6* | 7 | 8 | 9 | 10 | 11 | 12 | 13 | 14 |
|---|---|---|---|---|---|---|---|---|---|---|
| Huile** (g) | Colza | | 3,5 | 3,5 | | | | | | |
| | Soja | | | | 3,5 | 3,5 | | | | |
| | Exsol D 80 | | | | | | 3,5 | 3,5 | | |
| | Isopar M | | | | | | | | 3,5 | 3,5 |
| Tensio-actifs** (g) | Cemulsol NP 4 | | 1,5 | | | | 1 | 0,75 | 1 | 0,75 |
| | Cemulsol NP 17 | | | | | | 0,5 | 0,75 | 0,5 | 0,75 |
| | Cemulsol R 5 | | | | 1,35 | | | | | |
| | Cemulsol T | | | | 0,15 | | | | | |
| | Cemulsol NP 5 | | | | | | 1,5 | | | |
| | Cemulsol AS 5 | | | 1,5 | | | | | | |
| pH | | 6,75 | 6,2 | 6,5 | 6,55 | 6,35 | 6,3 | 5,75 | 6 | 5,9 |
| Viscosité *** (mPa.s) | | 9.600 | 11.700 | 7.400 | 8.700 | 11.100 | 7.000 | 10.500 | 12.900 | 12.900 |
| Sucre consommé g/l | | 45,6 | 49,5 | 60,5 | 47,2 | 48,4 | 54,5 | 63,3 | 62,2 | 67,1 |
| Polymère g/kg | | 32,4 | 41,3 | 33,8 | 35 | 36 | 35,9 | 41,2 | 41,2 | 42,8 |
| Rdt/sucre consommé | | 71 | 83 | 89 | 74 | 74 | 66 | 65 | 66 | 64 |

\* Témoin — Pas d'huile
\*\* Exsol D80® — Hydrocarbures aliphatiques Esso Chimie
Isopar M®: Isoparaffines — Esso Chimie
Cemulsol® NP4, NP5, NP17: nonylphénols éthoxylés — Société SFOS
Cemulsol® R5, AS5, T: alcools oléocétyliques éthoxylés — Société SFOS
\*\*\* Brookfield, 20°C, aig. n° 4, 30 t/mn

EP 0 187 092 B1

# EP 0 187 092 B1

**Revendications**

1. Procédé de production de polysaccharides épaississants par fermentation d'un milieu nutritif aqueux, contenant à titre de source hydrocarbonée de 10 à 200 g/l d'un hydrate de carbone, au moyen de microorganismes caractérisé en ce que l'on disperse une huile dans le milieu aqueux de manière à former une émulsion huile-dans-eau au sein de laquelle est réalisée la fermentation.

2. Procédé selon la revendication 1 caractérisé en ce que la proportion de phase huileuse représente moins de 30% et de préférence 1 à 18% en poids de l'émulsion.

3. Procédé selon la revendication 2 caractérisé en ce que la proportion de phase huileuse représente 3 à 16% en poids de l'émulsion.

4. Procédé selon l'une des revendications 1 à 3 caractérisé en ce que l'émulsion huile dans eau est stabilisée à l'aide d'au moins un agent tensio-actif non ionique.

5. Procédé selon la revendication 4 caractérisé en ce que l'agent tensio-actif ou le mélange d'agents tensio-actifs a une valeur HLB inférieure à 11 et de préférence comprise entre 6 et 11.

6. Procédé selon l'une des revendications 1 à 5 caractérisé en ce que le microorganisme est du genre Xanthomonas.

7. Procédé de préparation de polysaccharides épaississants par fermentation d'un milieu nutritif aqueux, contenant à titre de source hydrocarbonée de 10 à 200 g/l d'un hydrate de carbone, au moyen de microorganismes caractérisé en ce que la fermentation est réalisée au sein d'une émulsion huile dans eau contenant moins de 30% en poids de phase huileuse par rapport au poids de l'émulsion, puis après production du polysaccharide, l'émulsion est concentrée par élimination d'eau jusqu'à obtention d'une émulsion contenant 8 à 60% en poids de polysaccharide.

8. Utilisation des émulsions préparées par le procédé selon l'une des revendications 1 à 7 pour la préparation de fluides aqueux viscosifiés.

9. Utilisation selon la revendication 8 pour la préparation de fluides destinés au traitement des gisements pétroliers, en particulier à la récupération assistée du pétrole.

**Patentansprüche**

1. Verfahren zur Herstellung von Verdickungspolysachariden durch Fermentation eines wässrigen Nährmediums durch Mikroorganismen, das als Kohlenstoffquelle 10 bis 200 g/l eines Kohlenhydrates enthält, dadurch gekennzeichnet, daß man ein Öl in dem wässrigen Milieu zur Bildung einer Öl-in-Wasser-Emulsion dispergiert, in der die Fermentation durchgeführt wird.

2. Verfahren nach Anspruch 1, dadurch gekennzeichnet, daß der Anteil der ölhaltigen Phase weniger als 30 Gew.-% und vorzugsweise 1 bis 18 Gew.-% der Emulsion beträgt.

3. Verfahren nach Anspruch 2, dadurch gekennzeichnet, daß der Anteil der ölhaltigen Phase 3 bis 16 Gew.-% der Emulsion beträgt.

4. Verfahren nach einem der Ansprüche 1 bis 3, dadurch gekennzeichnet, daß die Ö'-in-Wasser-Emulsion mit Hilfe mindestens eines nichtionischen, oberflachenaktiven Mittels stabilisiert wird.

5. Verfahren nach Anspruch 4, dadurch gekennzeichnet, daß das oberflächenaktive Mittel oder das Gemisch aus oberflächenaktiven Mitteln einen HLB-Wert kleiner als 11 und vorzugsweise zwischen 6 und 11 hat.

6. Verfahren nach einem der Ansprüche 1 bis 5, dadurch gekennzeichnet, daß der Microorganismus von der Gattung der Xanthomonas ist.

7. Verfahren zur Herstellung von Verdickungspolysacchariden durch Fermantation eines wässrigen Nährmediums durch Microorganismen, das als Kohlenstoffquelle 10 bis 200 g/l eines Kohlenhydrates enthält, dadurch gekennzeichnet, daß die Fermentation in einer Öl-in-Wasser-Emulsion erfolgt, die weniger als 30 Gew.-% ölhaltige Phase im Verhältnis zum Emulsionsgewicht enthält, und anschliessend nach der Produktion des Polysaccharids die Emulsion durch Entfernung des Wassers bis zum Erreichen einer 8 bis 60 Gew.-% Polysaccharide enthaltenden Emulsion konzentriert wird.

8. Verwendung der durch das Verfahren nach einem der Ansprüche 1 bis 7 hergestellten Emulsionen zur Herstellung von wässrigen viskosen Fluiden.

9. Verwendung nach Anspruch 8 zur Herstellung von Flüssigkeiten, die zum Einsatz in Erdölfeldern bestimmt sind, insbesondere zur Unterstützung der Erdölgewinnung.

**Claims**

1. Process for producing thickening polysaccharides by fermentation of an aqueous nutrient medium containing from 10 to 200 g/l of a carbohydrate as a carbon source, by means of microorganisms, characterized in that an oil is dispersed in the aqueous medium so as to form an oil-in-water emulsion within which the fermentation is carried out.

2. Process according to Claim 1, characterized in that the proportion of oil phase represents less than 30%, an preferably 1 to 18%, by weight of the emulsion.

3. Process according to Claim 2, characterized in that the proportion of oil phase represents 3 to 16% by weight of the emulsion.

9

4. Process according to one of Claims 1 to 3, characterized in that the oil-in-water emulsion is stabilized with the aid of at least one nonionic surfactant.

5. Process according to Claim 4, characterized in that the surfactant or mixture of surfactants has an HLB value of less than 11, and preferably between 6 and 11.

6. Process according to one of Claims 1 to 5, characterized in that the microorganism is of the genus Xanthomonas.

7. Process for preparing thickening polysaccharides by fermentation of an aqueous nutrient medium containing from 10 to 200 g/l of a carbohydrate as a carbon source, by means of microorganisms, characterized in that the fermentation is carried out within an oil-in-water emulsion containing less than 30% by weight of oil phase relative to the weight of the emulsion and then, after production of the polysaccharide, the emulsion is concentrated by removing water until an emulsion containing 8 to 60% by weight of polysaccharide is obtained.

8. Use of the emulsions prepared by the process according to one of Claims 1 to 7 for preparing aqueous fluids having enhanced viscosity.

9. Use according to Claim 8 for preparing fluids intended for the treatment of oil deposits, especially for enhanced oil recovery.